# EUROPÄISCHE PATENTSCHRIFT

(11) **EP 1 474 414 B1**
(45) Veröffentlichungstag und Bekanntmachung des Hinweises auf die Patenterteilung: **12.12.2007**
(21) Anmeldenummer: 03706373.2
(22) Anmeldetag: 23.01.2003
(51) Int. Cl.: C07D 335/10, A61K 31/382, A61P 25/28

(54) **DIHYDRO-THIA-PHENANTHREN-CARBONYL-GUANIDINE, VERFAHREN ZU IHRER HERSTELLUNG, IHRE VERWENDUNG ALS MEDIKAMENT ODER DIAGNOSTIKUM**
DIHYDRO-THIA-PHENANTHRENE-CARBONYL-GUANIDINES, METHOD FOR THE PRODUCTION THEREOF, USE THEREOF AS A MEDICAMENT OR DIAGNOSTIC REAGENT
DIHYDRO-THIA-PHENANTHRENE-CARBONYL-GUANIDINES, PROCEDE DE PRODUCTION DESDITES SUBSTANCES ET LEUR UTILISATION EN TANT QUE MEDICAMENT OU PRODUIT DE DIAGNOSTIC, ET MEDICAMENT CONTENANT LESDITES SUBSTANCES

(30) Priorität: 07.02.2002 DE 10204989
(43) Veröffentlichungstag der Anmeldung: 10.11.2004
(73) Patentinhaber: Sanofi-Aventis Deutschland GmbH, 65929 Frankfurt am Main (DE)
(72) Erfinder: KLEEMANN, Heinz-Werner, 65474 Bischofsheim (DE); BELOW, Peter, 60529 Frankfurt (DE)
(86) Internationale Anmeldenummer: PCT/EP2003/000653
(87) Internationale Veröffentlichungsnummer: WO 2003/066620

(56) Entgegenhaltungen:
- GB-A- 2 171 996
- CHEMICAL ABSTRACTS, vol. 105, no. 74, 1986 Columbus, Ohio, US; abstract no. 70248x, Seite 653; Spalte 2; XP002239741 & JP 60 262887 A (MITSUBISHI CHEMICAL UND.) 26. Dezember 1985 (1985-12-26)

## Beschreibung

Dihydro-thia-phenanthren-carbonyl-guanidine, Verfahren zu ihrer Herstellung, ihre Verwendung als Medikament oder Diagnostikum sowie sie enthaltendes Medikament Die Erfindung betrifft Dihydro-thia-phenanthren-carbonyl-guanidine der Formel I 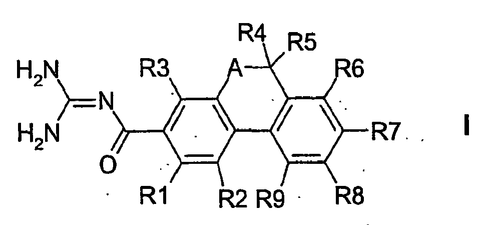 in welcher bedeuten:
- R(1) und R(3): unabhängig voneinander Wasserstoff, Alkyl mit 1, 2, 3 oder 4 C-Atomen, Alkoxy mit 1, 2, 3 oder 4 C-Atomen, F, Cl, Br, I, CN, NR(10)R(11),
-Oₚ-(CH₂)ₙ-(CF₂)ₓ-CF₃ oder -(SOₘ)ₚ-(CH₂)ₙ-(CF₂)ₓ-CF₃;
R(10) und R(11) unabhängig voneinander Wasserstoff, Alkyl mit 1, 2, 3 oder 4 C-Atomen oder -(CH₂)ₙ-(CF₂)ₓ-CF₃;
m Null, 1 oder 2;
n Null, 1, 2, 3, 4, 5 oder 6;
x und p unabhängig voneinander Null oder 1;
- R(2): Wasserstoff, F, Cl, Br, I, CN, Alkyl mit 1, 2, 3 oder 4 C-Atomen, Methoxy, Cycloalkyl mit 3, 4, 5, 6, 7 oder 8 C-Atomen,
- R(4) und R(5): unabhängig voneinander Wasserstoff, Alkyl mit 1, 2, 3 oder 4 C-Atomen;
- R(6), R(7), R(8) und R(9): unabhängig voneinander Wasserstoff, Alkyl mit 1, 2, 3 oder 4 C-Atomen; Alkoxy mit 1, 2, 3 oder 4 C-Atomen, F, Cl, Br, I, CN, NR(12)R(13), -O_{q}-(CH₂)ᵣ-(CF₂)ₛ-CF₃ oder -(SO_{w})ₜ-(CH₂)ᵤ-(CF₂)ᵥ-CF₃;
R(12) und R(13) unabhängig voneinander Wasserstoff, Alkyl mit 1, 2, 3 oder 4 C-Atomen;
w Null, 1 oder 2;
r und u Null, 1, 2, 3, 4, 5 oder 6;
q, s, t und v unabhängig voneinander Null oder 1;
oder
- R(6) und R(7) oder R(7) und R(8) oder R(8) und R(9): zusammen mit dem sie tragenden Phenylring ein Naphthalin-System;
- A: -S-, -SO- oder -SO₂-
sowie deren pharmazeutisch verträgliche Salze.

Bevorzugt sind Verbindungen der Formel I, in denen bedeuten:
- R(1) und R(3): unabhängig voneinander Wasserstoff, Methyl, Ethyl, Methoxy, Ethoxy, F, Cl, CN, NR(10)R(11), -Oₚ-(CH₂)ₙ-CF₃ oder -(SOₘ)ₚ-(CH₂)ₙ-CF₃;
R(10) und R(11) unabhängig voneinander Wasserstoff, Methyl, Ethyl oder -CH₂-CF₃;
m Null, 1 oder 2;
n Null, 1, 2 oder 3;
p unabhängig voneinander Null oder 1;
- R(2): Wasserstoff, F, Cl, CN, Alkyl mit 1, 2, 3 oder 4 C-Atomen, Methoxy, Cycloalkyl mit 3, 4, 5 oder 6 C-Atomen,
- R(4) und R(5): unabhängig voneinander Wasserstoff, Methyl oder Ethyl;
- R(6), R(7), R(8) und R(9): unabhängig voneinander Wasserstoff, Alkyl mit 1, 2, 3 oder 4 C-Atomen; Methoxy, Ethoxy, F, Cl, CN, NR(12)R(13), -O_{q}-(CH₂)ᵣ-CF₃ oder -(SO_{w})ₜ-(CH₂)ᵤ-CF₃;
R(12) und R(13) unabhängig voneinander Wasserstoff, Methyl oder Ethyl;
w Null, 1 oder 2;
r und u Null, 1, 2 oder 3;
q und t unabhängig voneinander Null oder 1;
oder
- R(6) und R(7) oder R(7) und R(8) oder R(8) und R(9): zusammen mit dem sie tragenden Phenylring ein Naphthalin-System;
- A: -S-, -SO- oder -SO₂-
sowie deren pharmazeutisch verträgliche Salze.

Besonders bevorzugt sind Verbindungen der Formel I, in denen bedeuten:
- R(1): Wasserstoff, Methyl, Ethyl, Methoxy, Ethoxy, F, Cl, NR(10)R(11), -Oₚ-(CH₂)ₙ-CF₃ oder -(SOₘ)ₚ-(CH₂)ₙ-CF₃;
R(10) und R(11) unabhängig voneinander Wasserstoff, Methyl, Ethyl oder -CH₂-CF₃;
m Null, 1 oder 2;
n, p unabhängig voneinander Null oder 1;
- R(2): Wasserstoff, F, Cl, Methyl, Cycloalkyl mit 3, 4, 5 oder 6 C-Atomen,
- R(3), R(4) und R(5): Wasserstoff;
- R(6), R(7), R(8) und R(9): unabhängig voneinander Wasserstoff, Methyl; Methoxy, Ethoxy, F, Cl, NR(12)R(13), -O_{q}-(CH₂)ᵣ-CF₃ oder -(SO_{w})ₜ-(CH₂)ᵤ-CF₃;
R(12) und R(13) unabhängig voneinander Wasserstoff, Methyl oder Ethyl;
w Null, 1 oder 2;
q, r, t und u unabhängig voneinander Null oder 1;
oder
- R(6) und R(7) oder R(7) und R(8) oder R(8) und R(9): zusammen mit dem sie tragenden Phenylring ein Naphthalin-System;
- A: -S-, -SO- oder -SO₂-
sowie deren pharmazeutisch verträgliche Salze.

Ganz besonders bevorzugt sind Verbindungen der Formel I, in denen bedeuten:
- R(1): Wasserstoff, Methyl, Methoxy, Ethoxy, Cl, NR(10)R(11), -O-CH₂-CF₃ oder -(SOₘ)ₚ-(CH₂)ₙ-CF₃;
R(10) und R(11) unabhängig voneinander Wasserstoff, Methyl, Ethyl oder-CH₂-CF₃;
m Null, 1 oder 2;
p Null oder 1 ;
- R(2): Wasserstoff, F, Cl oder Methyl;
- R(3), R(4) und R(5): Wasserstoff;
- R(6), R(7), R(8) und R(9): unabhängig voneinander Wasserstoff, Methyl; Methoxy, Ethoxy, F, Cl, -O-CH₂-CF₃ oder -(SO_{w})ₜ-(CH₂)ᵤ-CF₃;
w Null, 1 oder 2;
t und u unabhängig voneinander Null oder 1;
oder
- R(6) und R(7) oder R(7) und R(8) oder R(8) und R(9): zusammen mit dem sie tragenden Phenylring ein Naphthalin-System;
- A: -SO₂-
sowie deren pharmazeutisch verträgliche Salze.

Die Verbindungen der Formel I können bei entsprechender Substitution in stereoisomeren Formen vorliegen. Enthalten die Verbindungen der Formel I ein oder mehrere Asymmetriezentren, so können diese unabhängig voneinander die S-Konfiguration oder die R-Konfiguration aufweisen. Zur Erfindung gehören alle möglichen Stereoisomeren, z. B. Enantiomere oder Diastereomere, und Mischungen von zwei oder mehr stereoisomeren Formen, z. B. Enantiomeren und/oder Diastereomeren, in beliebigen Verhältnissen. Enantiomere z. B. gehören also in enantiomerenreiner Form, sowohl als links- als auch als rechtsdrehende Antipoden, und auch in Form von Mischungen der beiden Enantiomeren in unterschiedlichen Verhältnissen oder in Form von Racematen zu der Erfindung. Die Herstellung von einzelnen Stereoisomeren kann gewünschtenfalls durch Auftrennung eines Gemisches nach üblichen Methoden oder z. B. durch stereoselektive Synthese erfolgen. Bei Vorliegen von beweglichen Wasserstoffatomen umfasst die vorliegende Erfindung auch alle tautomeren Formen der Verbindungen der Formel I.

Die bezeichneten Alkylreste können geradkettig oder verzweigt sein.
Die Erfindung betrifft weiterhin ein Verfahren zur Herstellung der Verbindung I, dadurch gekennzeichnet, dass man eine Verbindung der Formel II 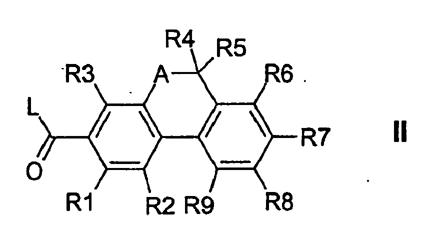 worin R(1) bis R(9) sowie A die angegebene Bedeutung besitzen und L für eine leicht nucleophil substituierbare Fluchtgruppe steht, mit Guanidin umsetzt.

Die aktivierten Säurederivate der Formel II; worin L eine Alkoxy-, vorzugsweise eine Methoxygruppe, eine Phenoxygruppe, Phenylthio-, Methylthio-, 2-Pyridylthiogruppe, einen Stickstoffheterocyclus, vorzugsweise "1-Imidazolyl, bedeutet, erhält man vorteilhaft in an sich bekannter Weise aus den zugrundeliegenden Carbonsäurechloriden (Formel II, L = Cl), die man ihrerseits wiederum in an sich bekannter Weise aus den zugrundeliegenden Carbonsäuren (Formel II, L = OH) beispielsweise mit Thionylchlorid herstellen kann.
Neben den Carbonsäurechloriden der Formel II (L = Cl) lassen sich auch weitere aktivierte Säurederivate der Formel II in an sich bekannter Weise direkt aus den zugrundeliegenden Benzoesäurederivaten (Formel II, L = OH) herstellen, wie die Methylester der Formel II mit L = OCH₃ durch Behandeln mit gasförmigem HCl in Methanol, die Imidazolide der Formel II durch Behandeln mit Carbonyldiimidazol [L = 1-Imidazolyl, Staab, Angew. Chem. Int. Ed. Engl. 1, 351 -367 (1962)], die gemischten Anhydride II mit Cl-COOC₂H₅ oder Tosylchlorid in Gegenwart von Triethylamin in einem inerten Lösungsmittel, wie auch die Aktivierungen von Benzoesäuren mit Dicyclohexylcarbodiimid (DCC) oder mit O-[(Cyano(ethoxycarbonyl)methylen)amino]-1,1,3,3-tetramethyluronium-tetrafluorborat ("TOTU") [Proceedings of the 21. European Peptide Symposium, Peptides 1990, Editors E. Giralt and D. Andreu, Escom, Leiden, 1991]. Eine Reihe geeigneter Methoden zur Herstellung von aktivierten Carbonsäurederivaten der Formel II sind unter Angabe von Quellenliteratur in J. March, Advanced Organic Chemistry, Third Edition (John Wiley & Sons, 1985), S. 350 angegeben.
Die Umsetzung eines aktivierten Carbonsäurederivates der Formel II mit Guanidin erfolgt in an sich bekannter Weise in einem protischen oder aprotischen polaren aber inerten organischen Lösungsmittel. Dabei haben sich bei der Umsetzung der Benzoesäuremethylester (II, L = OMe) mit Guanidin Methanol, Isopropanol oder THF von 20°C bis zur Siedetemperatur dieser Lösungsmittel bewährt. Bei den meisten Umsetzungen von Verbindungen II mit salzfreiem Guanidin wurde vorteilhaft in aprotischen inerten Lösungsmitteln wie THF, Dimethoxyethan, Dioxan gearbeitet. Aber auch Wasser kann unter Gebrauch einer Base wie beispielsweise NaOH als Lösungsmittel bei der Umsetzung von II mit Guanidin verwendet werden.
Wenn L = Cl bedeutet, arbeitet man vorteilhaft unter Zusatz eines Säurefängers, z. B. in Form von überschüssigen Guanidin zur Abbindung der Halogenwasserstoffsäure.

Zum Aufbau des Dihydro-thia-phenanthren-carbonsäure-Gerüstes geht man vorteilhaft von entsprechend substituierten Benzylsulfanylen, Phenylmethansulfinylen oder Phenylmethansulfonylen aus. Diese werden einer intramolekularen Aryl-Aryl-Kopplung unterzogen, wie sie prinzipiell bekannt ist (siehe Chem. Rev. 95 (7), 2457 (1995) oder "Metal-catalyzed Cross-coupling Reactions", Diederich, Francois; Stang, Peter J.; Editors Germany (1998) Publisher: (Wiley-VCH, Weinheim, Germany), 517) oder Tetrahedron (1998), 54(3/4), 263). Bevorzugt ist die Kopplung einer Boronsäure mit einem geeigneten Arylhalogenid, wie einem Arylchlorid, Arylbromid, Aryliodid oder einem geeigneten Arylester wie einem Aryl-mesylat oder Aryl-trifluormethansulfonat. Hierbei kann die Boronsäure-Funktion sowohl am Benzyl- als auch am Benzoesäure-Reaktionspartner eingeführt worden sein. Besonders bevorzugt ist auch die Verwendung von Bis(pinakolato)dibor wie in Tetrahedron Lett. (1997), 38(22), 3841 - 3844 beschrieben. In Formel III ist ein solches Ausgangsmaterial beschrieben, worin R(1) bis R(9) sowie A und L die angegebene Bedeutung besitzen und X und Y jeweils ein Halogen oder ein -O-SO₂CH₃ oder ein -O-SO₂CF₃ bedeuten. Bevorzugtes Katalysator-Metall ist Palladium, besonders bevorzugt in seinem Komplex Pd(dppf)₂. Die Reaktion wird in einem dipolar-aprotische Lösungsmittel durchgeführt, bevorzugt sind DMF oder DMA, bei einer Temperatur zwischen 0°C und dem Siedepunkt des Lösungsmittels, bevorzugt sind Temperaturen zwischen 40°C und 120°C.

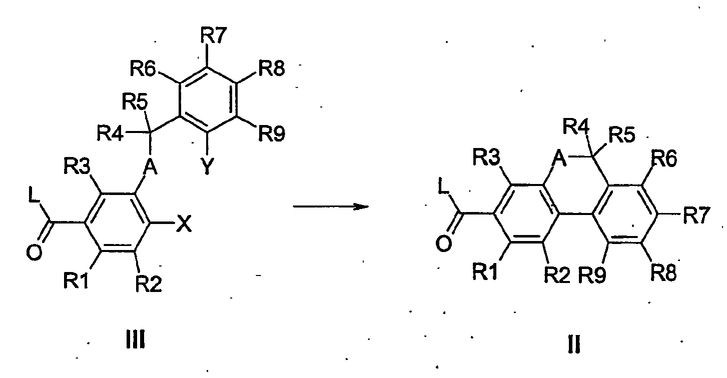

Die Derivate der allgemeinen Formel III werden bevorzugt aus 3-Mercaptobenzoesäure-Derivaten oder aus 3-Sulfino-benzoesäure-Derivaten der allgemeinen Formel IV mit aktivierten Benzyl-Derivaten der allgemeinen Formel V hergestellt:

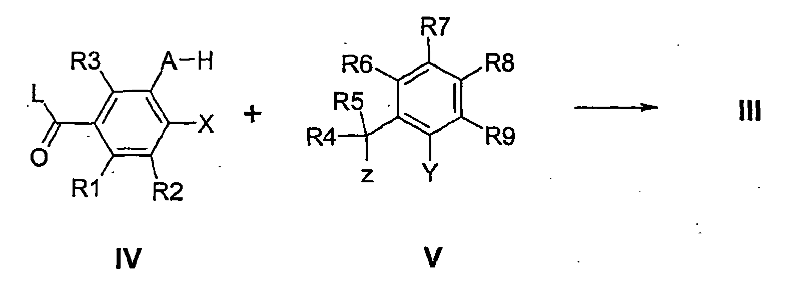

Hierbei handelt es sich bei Z um eine leicht nucleophil substituierbare Abgangsgruppe, wie zum Beispiel Chlor, Brom, Iod, Mesylat, Tosylat oder Trifluormethansulfonat. Die Derivate IV und V werden in einem geeigneten Lösungsmittel wie DMF, THF oder Acetonitril unter Verwendung einer Hilfsbase wie Triethylamin oder DIPEA bei einer Temperatur zwischen -20°C und dem Siedepunkt des Lösungsmittels, bevorzugt bei einer Temperatur zwischen 0°C und 40°C umgesetzt.

Aroylguanidine 1 sind im allgemeinen schwache Basen und können Säure unter Bildung von Salzen binden. Als Säureadditionssalze kommen Salze aller pharmakologisch verträglichen Säuren in Frage, beispielsweise Halogenide, insbesondere Hydrochloride, Lactate, Sulfate, Citrate, Tartrate, Acetate, Phosphate, Methylsulfonate, p-Toluolsulfonate.
Die Verbindungen 1 sind substituierte Acylguanidine.

Gegenüber den bekannten Verbindungen zeichnen sich die erfindungsgemäßen Verbindungen durch eine aüßerordentlich hohe Wirksamkeit in der Inhibition des Na⁺/H⁺-Austauschs aus.
Sie haben ebenso wie die bekannten Verbindungen keine unerwünschten und nachteiligen salidiuretischen, jedoch sehr gute antiarrhythmische Eigenschaften, wie sie beispielsweise zur Behandlung von Krankheitenwichtig sind, die bei Sauerstoffmangelerscheinungen auftreten. Die Verbindungen sind infolge ihrer pharmakologischen Eigenschaften als antiarrhythmische Arzneimittel mit cardioprotektiver Komponente zur Infarktprophylaxe und der Infarktbehandlung sowie zur Behandlung der angina pectoris hervorragend geeignet, wobei sie auch präventiv die pathophysiologischen Vorgänge beim Entstehen ischämisch induzierter Schäden, insbesondere bei der Auslösung ischämisch induzierter Herzarrhythmien, inhibieren oder stark vermindern. Wegen ihrer schützenden Wirkungen gegen pathologische hypoxische und ischämische Situationen können die erfindungsgemäßen Verbindungen der Formel.I infolge Inhibition des zellulären Na⁺/H⁺-Austauschmechanismus als Arzneimittel zur Behandlung aller akuten oder chronischen durch Ischämie ausgelösten Schäden oder dadurch primär oder sekundär induzierten Krankheiten verwendet werden. Dies betrifft ihre Verwendung als Arzneimittel für operative Eingriffe, z. B. bei Organ-Transplantationen, wobei die Verbindungen sowohl für den Schutz der Organe im Spender vor und während der Entnahme, zum Schutz entnommener Organe beispielsweise bei Behandlung mit oder deren Lagerung in physiologischen Badflüssigkeiten, wie auch bei der Überführung in den Empfängerorganismus verwendet werden können. Die Verbindungen sind ebenfalls wertvolle, protektiv wirkende Arzneimittel bei der Durchführung angioplastischer operativer Eingriffe beispielsweise am Herzen wie auch an peripheren Gefäßen. Entsprechend ihrer protektiven Wirkung gegen ischämisch induzierte Schäden sind die Verbindungen auch als Arzneimittel zur Behandlung von Ischämien des Nervensystems, insbesondere des ZNS, geeignet, wobei sie z.B. zur Behandlung des Schlaganfalls oder des Himödems geeignet sind. Darüber hinaus eignen sich die erfindungsgemäßen Verbindungen der Formel I ebenfalls zur Behandlung von Formen des Schocks, wie beispielweise des allergischen, cardiogenen, hypovolämischen und des bakteriellen Schocks.
Darüber hinaus zeichnen sich die erfindungsgemäßen Verbindungen der Formel I durch starke inhibierende Wirkung auf die Proliferationen von Zellen, beispielsweise der Fibroblasten- Zellproliferation und der Proliferation der glatten Gefäßmuskelzellen, aus. Deshalb kommen die Verbindungen der Formel I als wertvolle Therapeutika für Krankheiten in frage, bei denen die Zellproliferation eine primäre oder sekundäre Ursache darstellt, und können deshalb als Antiatherosklerotika, Mittel gegen diabetische Spätkomplikationen, Krebserkrankungen, fibrotische Erkrankungen wie Lungenfibrose, Leberfibrose oder Nierenfibrose, Organhypertrophien und - hyperplasien, insbesondere bei Prostatahyperplasie bzw. Prostatahypertrophie verwendet werden.

Die erfindungsgemäßen Verbindungen sind wirkungsvolle Inhibitoren des zellulären Natrium-Protonen-Antiporters (Na⁺/H⁺ -Exchanger), der bei zahlreichen Erkrankungen (Essentielle Hypertonie, Atherosklerose, Diabetes usw.) auch in solchen Zellen erhöht ist, die Messungen leicht zugänglich sind, wie beispielsweise in Erythrocyten, Thrombocyten oder Leukozyten. Die erfindungsgemäßen Verbindungen eignen sich deshalb als hervorragende und einfache wissenschaftliche Werkzeuge, beispielsweise in ihrer Verwendung als Diagnostika zur Bestimmung und Unterscheidung bestimmter Formen der Hypertonie, aber auch der Atherosklerose, des Diabetes, proliferativer Erkrankungen usw. Darüber hinaus sind die Verbindungen der Formel I für die präventive Therapie zur Verhinderung der Genese des Bluthochdrucks, beispielweise der essentiellen Hypertonie, geeignet.
Es wurde außerdem gefunden, dass Verbindungen der Formel I eine günstige Beeinflussung der Serumlipoproteine zeigen. Es ist allgemein anerkannt, dass für die Entstehung arteriosklerotischer Gefäßveränderungen, insbesondere der koronaren Herzkrankheit, zu hohe Blutfettwerte, sogenannte Hyperlipoproteinämien, einen wesentlichen Risikofaktor darstellen. Für die Prophylaxe und die Regression von atherosklerotischen Veränderungen kommt daher der Senkung erhöhter SerumLipoproteine eine außerordentliche Bedeutung zu. Neben der Reduktion des Serum-Gesamtcholesterins kommt der Senkung des Anteils spezifischer atherogener Lipidfraktionen dieses Gesamtcholesterins, insbesondere der low density Lipoproteine (LDL) und der very low density Lipoproteine (VLDL) eine besondere Bedeutung zu, da diese Lipidfraktionen einen atherogenen Risikofaktor darstellen. Dagegen wird den high density Lipoproteinen eine Schutzfunktion gegen die koronare Herzkrankheit zugeschrieben. Dementsprechend sollen Hypolipidämika in der Lage sein, nicht nur das Gesamtcholesterin, sondern insbesondere die VLDL und LDL-Serumcholesterinfraktionen zu senken.. Es wurde nun gefunden, dass Verbindungen der Formel I bezüglich der Beeinflussung der Serumlipidspiegel wertvolle therapeutisch verwertbare Eigenschaften zeigen. So erniedrigen sie signifikant die erhöhten Serum Konzentration von LDL und VLDL, wie sie beispielweise durch erhöhte diätetische Einnahme einer cholesterin- und lipidreichen Kost oder bei pathologischen Stoffwechselveränderungen, beispielsweise genetisch bedingten Hyperlipidämien zu beobachten sind. Sie können daher zur Prophylaxe und zur Regression von atherosklerotischen Veränderungen herangezogen werden, indem sie einen kausalen Risikofaktor ausschalten. Hierzu zählen nicht nur die primären Hyperlipidämien, sondern auch gewisse sekundäre Hyperlipidämien, wie sie z.B. beim Diabetes vorkommen. Darüber hinaus führen die Verbindungen der Formel I zu einer deutlichen Reduktion der durch Stoffwechselanomalien induzierten Infarkte und insbesondere zu einer signifikanten Verminderung der induzierten Infarktgröße und dessen Schweregrades. Weiterhin führen Verbindungen der Formel I zu einen wirksamen Schutz gegen durch Stoffwechselanomalien induzierter Endothelschädigungen. Mit diesem Schutz der Gefäße gegen das Syndrom der endothelialen Dysfunktion sind Verbindungen der Formel I wertvolle Arzneimittel zur Prävention und zur Behandlung koronarer Gefäßspasmen, der Atherogenese und der Atherosklerose, der linksventrikulären Hypertrophie und der dilatierten Kardiomyopathie, und thrombotischer Erkrankungen.
Die genannten Verbindungen finden deshalb vorteilhaft Verwendung zur Herstellung eines Medikaments zur Behandlung von Hypercholesterinämie; zur Herstellung eines Medikaments zur Prävention der Atherogenese; zur Herstellung eines Medikaments zur Prävention und Behandlung der Atherosklerose, zur Herstellung eines Medikaments zur Prävention und Behandlung von Krankheiten, die durch erhöhte Cholesterinspiegel ausgelöst werden, zur Herstellung eines Medikaments zur Prävention und Behandlung von Krankheiten; die durch endotheliale Dysfunktion ausgelöst werden, zur Herstellung eines Medikaments zur Prävention und Behandlung von atherosklerose-induzierter Hypertonie, zur Herstellung eines Medikaments zur Prävention und Behandlung von atherosklerose-induzierter Thrombosen, zur Herstellung eines Medikaments zur Prävention und Behandlung von Hypercholesterinämie- und endothelialer Dysfunktion induzierter ischämischer Schäden und postischämischer Reperfusionsschäden, zur Herstellung eines Medikaments zur Prävention und Behandlung von Hypercholesterinämie- und endothelialer Dysfunktion induzierter cardialer Hypertrophien, Cardiomyopathien und der congestiven Herzinsuffizienz (CHF), zur Herstellung eines Medikaments zur Prävention und Behandlung von Hypercholesterinämie- und endothelialer Dysfunktion induzierter koronarer Gefäßspasmen und myocardialer Infarkte, zur Herstellung eines Medikaments zur Behandlung der genannten Leiden in. Kombinationen mit blutdrucksenkenden Stoffen, bevorzugt mit Angiotensin Converting Enzyme (ACE)-Hemmem und Angiotensin-Rezeptorantagonisten, eine Kombination eines NHE-Inhibitors der Formel I mit einem blutfettspiegelsenkenden Wirkstoff, bevorzugt mit einem HMG-CoA-Reduktaseinhibitor (z.B. Lovastatin oder Pravastatin), wobei letzterer eine hypolipidämische Wirkung herbeiführt und dadurch die hypolipidämischen Eigenschaften des NHE-Inhibitors der Formel steigert, erweist sich als günstige Kombination mit verstärkter Wirkung und vermindertem Wirkstoffeinsatz.
Beansprucht wird die Gabe von Natrium-Protonen-Austausch-Hemmern der Formel I als neuartige Arzneimittel zur Senkung erhöhter Blutfettspiegel, sowie die Kombination von Natrium-Protonen-Austausch-Hemmern mit blutdrucksenkenden und/oder hypolipidämisch wirkenden Arzneimitteln.

Beansprucht wird außerdem die Gabe von Natrium-Protonen-Austausch-Hemmern der Formel I, sowie die Kombination von Natrium-Protonen-Austausch-Hemmern mit blutdrucksenkenden Medikamenten, insbesondere mit ACE-Hemmern (zum Beispiel Ramipril) sowie mit Angiotensin-Rezeptorantagonisten (zum Beispiel Losartan) als neuartige Arzneimittel zur Behandlung der CHF.

Arzneimittel, die eine Verbindung I enthalten, können dabei oral, parenteral, intravenös, rektal oder durch Inhalation appliziert werden, wobei die bevorzugte Applikation von dem jeweiligen Erscheinungsbild der Erkrankung abhängig ist. Die Verbindungen 1 können dabei allein oder zusammen mit galenischen Hilfsstoffen zur Anwendung kommen, und zwar sowohl in der Veterinär- als auch in der Humanmedizin.
Welche Hilfsstoffe für die gewünschte Arzneimittelformulierung geeignet sind, ist dem Fachmann auf Grund seines Fachwissens geläufig. Neben Lösemitteln, Gelbildnem, Suppositorien-Grundlagen, Tablettenhilfsstoffen, und anderen Wirkstoffträgem können beispielsweise Antioxidantien, Dispergiermittel, Emulgatoren, Entschäumer, Geschmackskorrigentien, Konservierungsmittel, Lösungsvermittler oder Farbstoffe verwendet werden.
Für eine orale Anwendungsform werden die aktiven Verbindungen mit den dafür geeigneten Zusatzstoffen, wie Trägerstoffen, Stabilisatoren oder inerten Verdünnungsmittel vermischt und durch die üblichen Methoden in die geeigneten Darreichungsformen gebracht, wie Tabletten, Dragees, Steckkapseln, wäßrige, alkoholische oder ölige Lösungen. Als inerte Träger können z. B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie Sonnenblumenöl oder Lebertran.
Zur subkutanen oder intravenösen Applikation werden die aktiven Verbindungen, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z. B. in Frage: Wasser, physiologische Kochsalzlösung oder Alkohole, z. B. Ethanol, Propanol, Glycerin, daneben auch Zuckerlösungen wie Glucose- oder Mannitlösungen, oder auch eine Mischung aus den verschiedenen genannten Lösungsmitteln.
Als pharmazeutische Formulierung für die Verabreichung in Form von Aerosolen oder Sprays sind geeignet z. B. Lösungen, Suspensionen oder Emulsionen des Wirkstoffes der Formel I in einem pharmazeutisch unbedenklichen Lösungsmittels, wie insbesondere Ethanol oder Wasser, oder einem Gemisch solcher Lösungsmittel.
Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gew.-%.
Die Dosierung des zu verabreichenden Wirkstoffs der Formel I und die Häufigkeit der Verabreichung hängen von der Wirkstärke und Wirkdauer der verwendeten Verbindungen ab; außerdem auch von Art und Stärke der zu behandelnden Krankheit sowie von Geschlecht, Alter, Gewicht und individueller Ansprechbarkeit des zu behandelnden Säugers.
Im Durchschnitt beträgt die tägliche Dosis einer Verbindung der Formel I bei einem etwa 75 kg schweren Patienten mindestens 0,001 mg/kg, vorzugsweise 0,01 mg/kg, bis höchstens 10 mg/kg, vorzugsweise bis höchstens 1 mg/kg Körpergewicht. Bei akuten Ausbrüchen der Krankheit, etwa unmittelbar nach Erleiden eines Herzinfarkts, können auch noch höhere und vor allem häufigere Dosierungen notwendig sein, z. B. bis zu 4 Einzeldosen pro Tag: Insbesondere bei i.v. Anwendung, etwa bei einem Infarktpatienten auf der Intensivstation können bis zu 200 mg pro Tag und kg Körpergewicht notwendig werden.

### Liste der Abkürzungen:

- DIPEA: Diisopropylethylamin
- DMA: N,N-Dimethylacetamid
- DME: 1,2-Dimethoxyethan
- DMF: N,N-Dimethylformamid
- EE: Ethylacetat (EtOAc)
- eq.: Äquivalent
- MeOH: Methanol
- Pd(dppf)₂: [1,1'-Bis-(diphenylphosphino)-ferrocen]palladium(II)-chlorid-Methylenchlorid-Komplex (1:1)
- RT: Raumtemperatur
- Smp: Schmelzpunkt
- THF: Tetrahydrofuran

### Experimenteller Teil

Allgemeine Arbeitsvorschrift zur Synthese von Dihydro-thia-phenanthren-carbonylguanidinen

### Stufe 1) 4-Brom-5-chlorsulfonyl-2-methyl-benzoesäure-methylester

12 g 4-Brom-5-chlorsulfonyl-2-methyl-benzoesäure (J.Med.Chem. 1997, 40, 2017) wurden mit 20 ml Thionylchlorid für 8 Stunden unter Feuchtigkeitsausschluß am Rückfluß gekocht. Das überschüssige Thionylchlorid wurde am Rotationsverdampfer im Vakuum entfernt, der Rückstand mit ca. 50 ml trockenem Toluol aufgenommen und nochmals evaporiert. Das rohe Säurechlorid wurde in 25 ml wasserfreiem Toluol gelöst, 1.7 ml MeOH zugegeben und 2 h bei 50°C gerührt. Anschließend wurden weitere 1.7 ml MeOH zugegeben und noch 4 h bei 50°C gerührt. Das Reaktionsgemisch wurde mit 200 ml EE verdünnt und mit 100 ml einer gesätigten wäßrigen NaHCO₃-Lösung gewaschen. Über Na₂SO₄ wurde getrocknet und die Solventien im Vakuum entfernt. Man erhielt 11.0 g eines blassgelben Öls, das ohne weitere Reinigung verwendet wurde.

### Stufe 2) 2-Brom-5-methoxycarbonyl-4-methyl-benzolsulfnsäure

550 mg Na₂SO₃ wurden in 2 ml Wasser gelöst und bei 70°C eine Lösung von 337 mg 4-Brom-5-chlorsulfonyl-2-methyl-benzoesäure-methylester in 2 ml DME zugetropft. Während des Zutropfens wurde die Lösung schwach sauer (pH=5). 2.5 h wurde bei 70°C nachgerührt, man ließ abkühlen und stellte mit einer wäßrigen HCl-Lösung auf pH=1-2. Mit 50 ml EE wurde verdünnt und mit 50 ml einer gesättigten wäßrigen NaCl-Lösung gewaschen. Über Na₂SO₄ wurde getrocknet und die Solventien im Vakuum entfernt. Man erhielt 228 mg eines blaßgelben Öls, das ohne weitere Reinigung verwendet wurde.

### Stufe 3) 4-Brom-5-(2-brom-phenylmethansulfonyl)-2-methyl-benzoesäure-methylester

150 mg (0.51 mmol) 2-brom-5-methoxycarbonyl-4-methyl-benzolsulfinsäure (Stufe 2) wurden in 1.5 ml DMF gelöst. Dazu gab man 128 mg (0.51 mmol) 2-Brombenzylbromid in 0.5 ml DMF gelöst und 0.1 ml (0.56 mMol) DIPEA und ließ bei Raumtemperatur für 16 Stunden unter Feuchtigkeitsausschluß rühren. Die Reaktionslösung wurde filtriert, mit 20 ml EE verdünnt, mit 20 ml 1n Salzsäure und dann 20 ml 5%-iger Kochsalzlösung gewaschen. Die organische Phase wurde über eine Trockenkartusche (wasserfreies Natriumsulfat) gepresst, die Kartusche mit 5 ml EE nachgewaschen. Das Filtrat wurde evaporiert. Das Rohprodukt wurde durch präparative HPLC gereinigt.

Entsprechend der allgemeinen Reaktionsgleichung 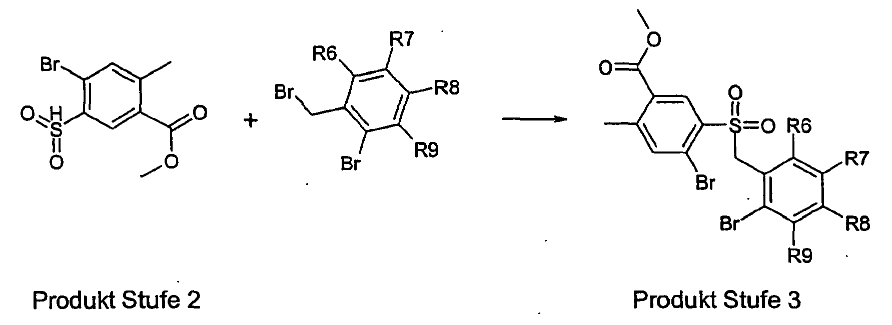 wurden analog die folgenden Produkte hergestellt:

| Nr. | Benzylbromid | Produkt |
|---|---|---|
| 2 | 1-Brom-2-(brommethyl)naphthalin | 4-Brom-5-(1-brom-naphthalin-2-ylmethansulfonyl)-2-methyl-benzoesäure - methyl ester |
| 3 | 2-Brom-4-methylbenzyl bromid | 4-Brom-5-(2-brom-4-methyl-phenylmethansulfonyl)-2-methyl-benzoesäure-methylester |
| 4 | 1-Brom-2-brommethyl-4-chlor-benzol | 4-Brom-5-(2-brom-5-chlor-phenylmethansulfonyl)-2-methyl-benzoesäure- methylester |
| 5 | 2-Brom-1-brommethyl-4-fluor-benzol | 4-Brom-5-(2-brom-4-fluor-phenylmethansulfonyl)-2-methyl-benzoesäure-methylester |
| 6 | 1-Brom-2-brommethyl-3-fluor-benzol | 4-Brom-5-(2-brom-6-fluor-phenylmethansulfonyl)-2-methyl-benzoesäure-methylester |
| 7 | 2-Brom-1-brommethyl-4-chlor-benzol | 4-Brom-5-(2-brom-4-chlor-phenylmathansulfonyl)-2-methyl-benzoesäure-methylester |
| 8 | 1-Brom-2-brommethyl-4-methoxy-benzol | 4-Brom-5-(2-brom-5-methoxy-phenylmethansulfonyl)-2-methyl-benzoesäure-methylester |
| 9 | 1-Brom-2-brommethyl-3-chlor-benzol | 4-Brom-5-(2-brom-6-chlor-phenylmethansulfonyl)-2-methyl-benzoesäure-methylester |
| 10 | 2-Brom-1-brommethyl-3-methyl-benzol | 4-Brom-5-(2-brom-3-methyl-phenylmethansulfonyl)-2-methyl-benzoesäure-methyl-ester |
| 11 | 1-Brom-2-brommethyl-4-methyl-benzol | 4-Brom-5-(2-brom-5-methyl-phenylmethansulfonyl)-2-methyl-benzoesäure-methyl-ester |

Die eingesetzten Benzylbromide wurden, soweit nicht käuflich, entweder aus den entsprechenden Methylaromaten durch radikalische Bromierung mit N-Bromsuccinimid oder aus den Benzylalkoholen durch Umsetzung mit wässriger HBr oder Methansulfonsäurechlorid/Triethylamin, gefolgt von Tetrabutylammoniumbromid hergestellt.
Die Rohprodukte wurden mit Acetonitril/Wasser = 9:1 (1 ml) eventuell unter Zusatz von 0,2 ml DMF verrührt, über Kartuschen abgesaugt und mit Acetonitril/Wasser = 9:1 (0,5 ml) nachgewaschen. Die ausgefallenen Feststoffe wurden im Vakuumtrockenschrank bei 50°C getrocknet und die Reinheiten waren laut HPLC/MS >80 %.Die Mutterlaugen wurden über präparative HPLC gereinigt, da sie noch einen großen Anteil Produkt enthielten.

### Stufe 4) 6-Methyl-9,9-dioxo-9,10-dihydro-9-thia-phenanthren-7-carbonsäure-methylester

68 mg (0,266 mmol) Bis(pinakolato)dibor, 71 mg (0,725 mmol) Kaliumacetat und 9 mg (0,012 mmol) Pd(dppf)₂ wurden in 2 ml DMA vorgelegt. Dazu gab man 112 mg (0,242 mmol) 4-Brom-5-(2-brom-phenylmethansulfonyl)-2-methyl-benzoesäure-methylester (Stufe 3) in 4 ml DMA gelöst und rührte bei 80 °C über Nacht unter Schutzgas. Die Reaktionslösung wurde über Kieselgel filtriert und mit 20 ml EE nachgewaschen. Die organische Phase wurde mit Wasser und 5 %-iger Kochsalzlösung gewaschen, über wasserfreiem Natriumsulfat getrocknet und im Vakuum evaporiert. Das Rohprodukt wurde durch präparative HPLC gereinigt.
Entsprechend der allgemeine Reaktionsgleichung 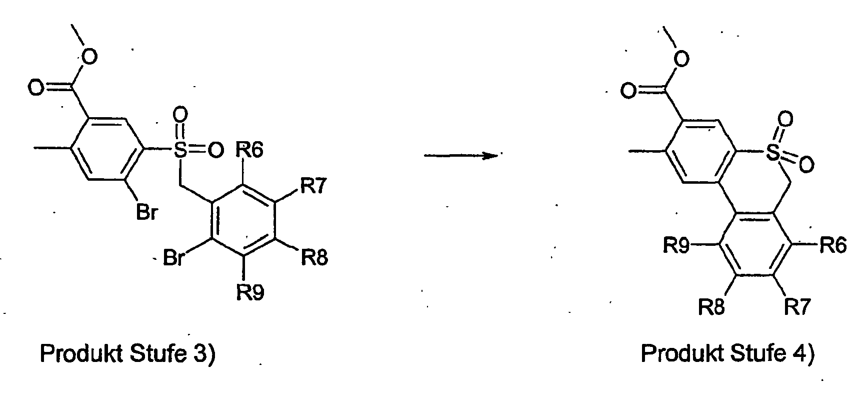 wurden analog die folgenden Produkte hergestellt:

| Nr. | Produkt |
|---|---|
| 2 | 2-Methyl-5,5-dioxo-5,6-dihydro-5-thia-benzo[c]phenanthren-3-carbonsäure-methyl-ester |
| 3 | 3,6-Dimethyl-9,9-dioxo-9,10-dihydro-9-thia-phenanthren-7-carbonsäure-methylester |
| 4 | 2-Chlor-6-methyl-9,9-dioxo-9,10-dihydro-9-thia-phenanthren-7-carbonsäure-methylester |
| 5 | 3-Fluor-6-methyl-9,9-dioxo-9,10-dihydro-9-thia-phenanthren-7-carbonsäure-methylester |
| 6 | 1-Fluor-6-methyl-9,9-dioxo-9,10-dihydro-9-thia-phenanthren-7-carbonsäure-methylester |
| 7 | 3-Chlor-6-methyl-9,9-dioxo-9,10-dihydro-9-thia-phenanthren-7-carbonsäure-methylester |
| 8 | 2-Methoxy-6-methyl-9,9-dioxo-9,10-dihydro-9-thia-phenanthren-7-carbonsäure-methylester |
| 9 | 1-Chlor-6-methyl-9,9-dioxo-9,10-dihydro-9-thia-phenanthren-7-carbonsäure-methylester |
| 10 | 4,6-Dimethyl-9,9-dioxo-9,10-dihydro-9-thia-phenanthren-7-carbonsäure-methylester |
| 11 | 2,6-Dimethyl-9,9-dioxo-9,10-dihydro-9-thia-phenanthren-7-carbonsäure-methylester |

### Stufe 5) Dihydro-thia-phenanthren-carbonyl-guanidine, allgemeine Vorschrift

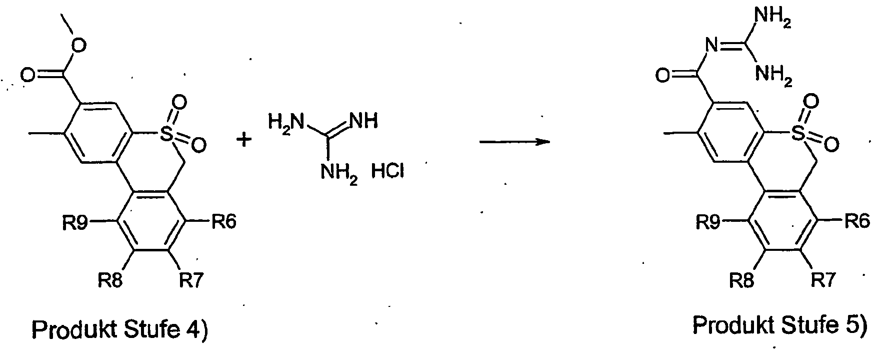

53 mg (0.5 mmol) Kalium-tert.-butylat wurden in 2 ml trockenem DMF suspendiert. Dazu gab man 50 mg (0.55 mmol) Guanidin-Hydrochlorid und rührte die Suspension unter Feuchtigkeitsausschluss 30 Min. bei RT. Anschließend gab man 0.1 mmol des Methylesters aus Stufe 4 gelöst in 1 ml DMF zu und ließ über Nacht bei RT rühren. Die ausgefallenen Salze wurden abfiltriert und das Filtrat wurde direkt über präparative HPLC gereinigt (Säulenmaterial Merck Supersphere RP18e, Acetonitril/Wasser Gradient mit 0.1 % Ameisensäure als Puffer). Die erhaltenen Produkte wurden durch analytische HPLC/IVIS auf einer Agilent Serie 1100 Anlage charakterisiert.
Die Massendetektion wurde mit positiver Ionisation durchgeführt.

### Methode A:

| | | |
|---|---|---|
| Säule: | | MERCK LiChroCart 55-2 |
| Packung: | | PuroSpher STAR RP18 |
| Fluss: | | 0,75 ml/min |
| Temperatur: | | 40 °C |
| Gradient: | | |
| | Solvent A | Acetonitril/Wasser (90:10) + 0,5 % Ameisensäure |
| | Solvent B | Acetonitril/Wasser (10:90) + 0,5 % Ameisensäure |

| Zeit | Solv. B |
|---|---|
| [min] | [%] |
| 0.00 | 95.0 |
| 0.50 | 95.0 |
| 1.75 | 5.0 |
| 4.25 | 5.0 |
| 4.50 | 95.0 |
| 5.00 | 95.0 |
| 6.20 | STOP |

### Methode B:

| | | |
|---|---|---|
| Säule: | | YMC J'Sphere ODS H80 |
| Packung: | | 4 µ |
| Fluss: | | 1,0 ml/min |
| Temperatur: | | 30 °C |
| Gradient: | | |
| | Solvent A | /Wasser + 0,05 % Trifluoressigsäure |
| | Solvent B | Acetonitril |

| Zeit | Solv. B |
|---|---|
| [min] | [%] |
| 0.00 | 10.0 |
| 2.50 | 95.0 |
| 3.30 | 95.0 |
| 3.35 | 10.0 |
| 3.60 | STOP |

Nach der allgemeinen Arbeitsvorschrift zur Synthese von Dihydro-thia-phenanthren-carbonyl-guanidinen wurden die Titelverbindungen der Beispiele 1-11 synthetisiert:

### Beispiel 1: N-(6-Methyl-9,9-dioxo-9,10-dihydro-9-thia-phenanthren-7-carbonyl)-guanidinium-formiat

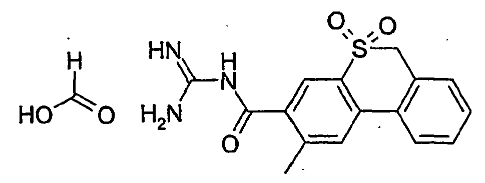

MS (ES) : 330 (M+1)⁺ Retentionszeit 2,384 min (220 nm, Methode A)

### Beispiel 2: N-(2-Methyl-5,5-dioxo-5,6-dihydro-5-thia-benzo[c]phenanthren-3-carbonyl)-guanidinium-formiat

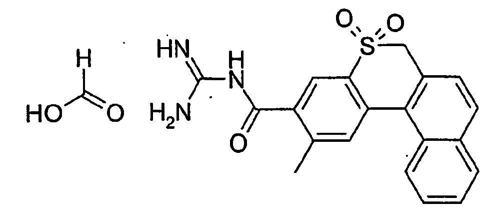

MS (ES): 380 (M+1)⁺ Retentionszeit 1,793 min (220 nm, Methode B)

### Beispiel 3: N-(3,6-Dimethyl-9,9-dioxo-9,10-dihydro-9-thia-phenanthren-7-carbonyl)-guanidinium-formiat

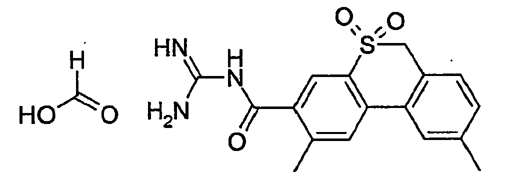

MS ( ) : 344 (M+1)⁺ Retentionszeit 2,411 min (220 nm, Methode A)

### Beispiel 4: N-(2-Chlor-6-methyl-9,9-dioxo-9,10-dihydro-9-thia-phenanthren-7-carbonyl)- guanidinium-formiat

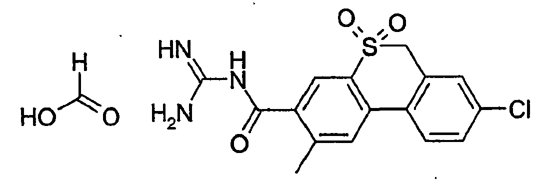

MS (ES) : 364 (M+1)⁺ Retentionszeit 2,390 min (220 nm, Methode A)

### Beispiel 5: N-(3-Fluor-6-methyl-9,9-dioxo-9,10-dihydro-9-thia-phenanthren-7-carbonyl)-guanidinium-formiat

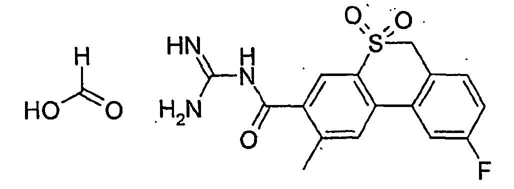

MS ES) : 348 (M+1)⁺ Retentionszeit 2,215 min (220 nm, Methode A)

### Beispiel 6: N-(1-Fluor-6-methyl-9,9-dioxo-9,10-dihydro-9-thia-phenanthren-7-carbonyl)-guanidinium-formiat

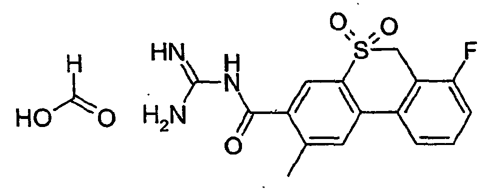

MS (ES) : 348 (M+1)⁺ Retentionszeit 2,218 min (220 nm, Methode A)

### Beispiel 7: N-(3-Chlor-6-methyl-9,9-dioxo-9,10-dihydro-9-thia-phenanthren-7-carbonyl)- guanidinium-formiat

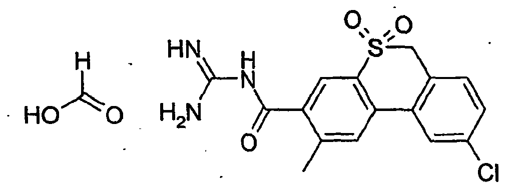

MS (ES) : 364 (M+1)⁺ Retentionszeit 2,394 min (220 nm, Methode A)

### Beispiel 8: N-(2-Methoxy-6-methyl-9,9-dioxo-9,10-dihydro-9-thia-phenanthren-7-carbonyl)- guanidinium-formiat

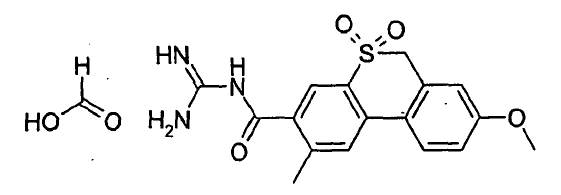

MS (ES) : 360 (M+1)⁺ Retentionszeit 2,271 min (220 nm, Methode A)

### Beispiel 9: N-(1-Chlor-6-methyl-9,9-dioxo-9,10-dihydro-9-thia-phenanthren-7-carbonyl)- guanidinium-formiat

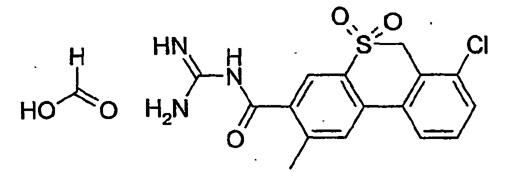

MS (ES) : 364 (M+1)⁺ Retentionszeit 2,358 min (220 nm, Methode A)

### Beispiel 10: N-(4,6-Dimethyl-9,9-dioxo-9,10-dihydro-9-thia-phenanthren-7-carbonyl)-guanidinium-formiat

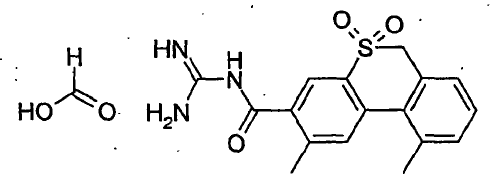

MS (ES) : 344 (M+1)⁺ Retentionszeit 2,302 min (220 nm, Methode A)

### Beispiel 11: N-(2,6-Dimethyl-9,9-dioxo-9,10-dihydro-9-thia-phenanthren-7-carbonyl)-guanidinium-formiat

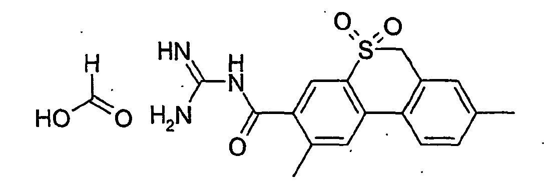

MS (ES) : 344 (M+1)⁺ Retentionszeit 2,671 min (220 nm, Methode A)

### Methode NHE-Hemmung Jansen

Die NHE-1 Hemmung IC₅₀ [nM] wurde wie folgt bestimmt:
FLIPR-Assay zur Bestimmung von NHE-1 Inhibitoren mittels Messung der pHi-Erholung in transfizierten Zelllinien, die den humanen NHE-1 exprimieren

Der Assay wird im FLIPR (Fluorescent imaging plate reader) mit schwarzwandigen 96-Well-Mikrotiterplatten mit klarem Boden durchgeführt. Die transfizierten Zelllinien, welche die verschiedenen NHE-Subtypen exprimieren (die parentale Zelllinie LAP-1 [erhalten von Prof. Pouysségur, Nizza] weist als Folge von Mutagenese und anschließender Selektion keine endogene NHE-Aktivität auf), werden am Vortag mit einer Dichte von -25.000 Zellen / Well.
[Das Wachstumsmedium der transfizierten Zellen (Iscove +10 % fötales Kälberserum) enthält zusätzlich G418 als Selektionsantibiotikum, um die Anwesenheit der transfizierten Sequenzen sicherzustellen.]

Der eigentliche Assay beginnt mit der Entfernung des Wachstumsmediums und Zugabe von 100 µl /Well Beladungspuffer (5 µM BCECF-AM [2',7'-Bis-(Carboxyethyl)-5-(und-6)-Carboxyfluorescein, Acetoxymethyl ester] in 20 mM NH₄Cl, 115 mM Cholinchloride, 1 mM MgCl₂, 1 mM CaCl₂, 5 mM KCl, 20 mM HEPES, 5 mM Glucose; pH 7,4 [mit KOH eingestellt]). Die Zellen werden dann 20 Minuten bei 37°C inkubiert. Diese Inkubation führt zur Beladung der Zellen mit dem fluoreszierenden Farbstoff, dessen Fluoreszenzintensität vom pHi abhängt, und mit NH₄Cl, was zu einer leichten Alkalinisierung der Zellen führt.

[Die nicht fluoreszierende Farbstoff-Vorstufe BCECF-AM ist als Ester membranpermeabel. Intrazellulär wird durch Esterasen der eigentliche Farbstoff BCECF freigesetzt, der nicht membranpermeabel ist.]

Nach dieser 20-minütigen Inkubation wird der Beladungspuffer, der NH₄Cl und freies BCECF-AM enthält, durch dreimaliges Waschen im Zellwasher (Tecan Columbus) mit jeweils 400 µl Waschpuffer (133,8 mM Choline chloride, 4,7 mM KCl, 1,25 mM MgCl₂, 1,25 mM CaCl₂, 0,97 mM K₂HPO₄, 0,23 mM KH₂PO₄, 5 mM HEPES, 5 mM Glucose; pH 7,4 [mit KOH eingestellt]) entfernt. Das in den Wells verbleibende Restvolumen beträgt 90 µl (50 -125 µl möglich). Dieser Waschschritt entfernt das freie BCECF-AM und führt als Folge der Entfernung der externen NH₄⁺-Ionen zu einer intrazellulären Ansäuerung (- pHi 6.3 - 6.4).
Da das Gleichgewicht von intrazellulärem NH₄⁺ mit NH₃ und H⁺ durch das Entfernen des extrazellulären NH₄⁺ und durch die nachfolgende, augenblicklich ablaufende Passage des NH₃ durch die Zellmembran gestört wird, führt der Waschprozess dazu, das intrazellulär H⁺ zurückbleibt, was die Ursache für die intrazelluläre Ansäuerung ist. Diese kann letztlich zum Zelltod führen, wenn sie lang genug anhält-An dieser Stelle ist es wichtig, dass der Waschpuffer natriumfrei (<1 mM) ist, da extrazelluläre Natrium-Ionen zu einer augenblicklichen Erholung des pHi durch die Aktivität der klonierten NHE-Isoformen führen würde.
Es ist ebenfalls wichtig, dass alle verwendeten Puffer (Beladungspuffer, Waschpuffer, Recoverypuffer) keine HCO₃-Ionen enthalten, da die Anwesenheit von Bicarbonat zur Aktivierung störender bicarbonatabhängiger pHᵢ-Regulationssysteme führen würde, die in der parentalen LAP-1 Zelllinie enthalten sind.

Die Mikrotiterplatten mit den angesäuerten Zellen werden dann (bis zu 20 Minuten nach der Ansäuerung) zum FLIPR transferiert. Im FLlPR wird der intrazelluläre Fluoreszenzfarbstoff durch Licht mit einer Wellenlänge von 488 nm, das von einem Argon-Laser erzeugt wird, angeregt, und die Messparameter (Laserleistung, Belichtungszeit und Blende der im FLIPR eingebauten CCD-Kamera) werden so gewählt, dass das durchschnittliche Fluoreszenzsignal pro Well zwischen 30000 und 35000 relativen Fluoreszenzeinheiten liegt.

Die eigentliche Messung im FLIPR beginnt damit, dass Software gesteuert alle zwei eine Aufnahme mit der CCD-Kamera gemacht wird. Nach zehn Sekunden wird die Erholung des intrazellulärer pHs durch Zugabe von 90 µl Recoverypuffer (133,8 mM NaCl, 4,7 mM KCl, 1,25 mM MgCl₂, 1,25 mM CaCl₂, 0,97 mM K₂HPO₄, 0,23 mM KH₂PO₄,10 mM HEPES, 5 mM Glucose; pH 7.4 [mit NaOH eingestellt]) mittels des im FLIPR eingebauten 96-Well-Pipettierers eingeleitet.
Als Positivkontrollen (100 % NHE-Aktivität) dienen Wells, denen reiner Recoverypuffer zugegeben wird, Negativkontrollen (0 %NHE-Aktivität) erhalten Waschpuffer. In allen anderen Wells wird Recoverypuffer mit der zweifach konzentrierten Testsubstanz hinzugegeben. Die Messung im FLIPR endet nach 60 Messpunkten (zwei Minuten).

Die Rohdaten werden in das Programm ActivityBase exportiert. Mit diesem Programm werden zunächst die NHE-Aktivitäten für jede getestete Substanzkonzentration und daraus die IC₅₀-Werte für die Substanzen berechnet. Da der Verlauf der pHi-Erholung nicht während des ganzen Experiments linear ist, sondern am Ende aufgrund abnehmender NHE-Aktivität bei höheren pHi-Werten abfällt, ist es wichtig, für die Auswertung der Messung den Teil auszuwählen, in dem die Fluoreszenzzunahme der Positivkontrollen linear ist.

| Beispiel | NHE-1 Hemmung IC₅₀ [nM] |
|---|---|
| 1 | 15.6 |
| 2 | 24.2 |
| 3 | 8.6 |
| 4 | 10.8 |
| 5 | 15.6 |
| 6 | 16.5 |
| 7 | 11.7 |
| 8 | 10.2 |
| 9 | 13.9 |
| 10 | 67.7 |
| 11 | 19.5 |

## Patentansprüche

1. Dihydro-thia-phenanthren-carbonyl-guanidine der Formel I 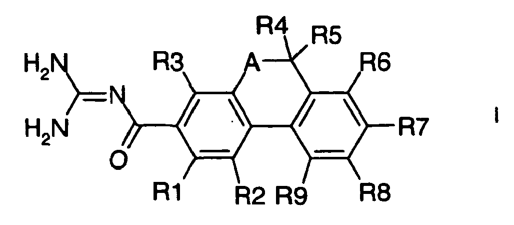 in welcher bedeuten:
R(1) und R(3) unabhängig voneinander Wasserstoff, Alkyl mit 1, 2, 3 oder 4 C-Atomen, Alkoxy mit 1, 2, 3 oder 4 C-Atomen, F, Cl, Br, I, CN, NR(10)R(11),
-Oₚ-(CH₂)ₙ-(CF₂)ₓ-CF₃ oder -(SOₘ)ₚ-(CH₂)ₙ-(CF₂)ₓ-CF₃;
R(10) und R(11) unabhängig voneinander Wasserstoff, Alkyl mit 1, 2, 3 oder 4 C-Atomen oder -(CH₂)ₙ-(CF₂)ₓ-CF₃;
m Null, 1 oder 2;
n Null, 1, 2, 3, 4, 5 oder 6;
x und p unabhängig voneinander Null oder 1;
R(2) Wasserstoff, F, Cl, Br, I, CN, Alkyl mit 1, 2, 3 oder 4 C-Atomen, Methoxy, Cycloalkyl mit 3, 4, 5, 6, 7 oder 8 C-Atomen,
R(4) und R(5) unabhängig voneinander Wasserstoff, Alkyl mit 1, 2, 3 oder 4 C-Atomen;
R(6), R(7), R(8) und R(9) unabhängig voneinander Wasserstoff, Alkyl mit 1, 2, 3 oder 4 C-Atomen; Alkoxy mit 1, 2, 3 oder 4 C-Atomen, F, Cl, Br, I, CN, NR(12)R(13), -O_{q}-(CH₂)ᵣ-(CF₂)ₛ-CF₃ oder -(SO_{w})ₜ-(CH₂)ᵤ-(CF₂)ᵥ-CF₃;
R(12) und R(13) unabhängig voneinander Wasserstoff, Alkyl mit 1, 2, 3 oder 4 C-Atomen;
w Null, 1 oder 2;
r und u Null, 1, 2, 3, 4, 5 oder 6;
q, s, t und v unabhängig voneinander Null oder 1;
oder
R(6) und R(7) oder R(7) und R(8) oder R(8) und R(9) zusammen mit dem sie tragenden Phenylring ein Naphthalin-System;
A -S-, -SO- oder -SO₂-
sowie deren pharmazeutisch verträgliche Salze.

2. Verbindungen der Formel I nach Anspruch 1, in der bedeuten:
R(1) und R(3) unabhängig voneinander Wasserstoff, Methyl, Ethyl, Methoxy, Ethoxy, F, Cl, CN, NR(10)R(11), -Oₚ-(CH₂)ₙ-CF₃ oder-(SOₘ)ₚ-(CH₂)ₙ-CF₃;
R(10) und R(11) unabhängig voneinander Wasserstoff, Methyl, Ethyl oder -CH₂-CF₃;
m Null, 1 oder 2;
n Null, 1, 2 oder 3;
p unabhängig voneinander Null oder 1;
R(2) Wasserstoff, F, Cl, CN, Alkyl mit 1, 2, 3 oder 4 C-Atomen, Methoxy, Cycloalkyl mit 3, 4, 5 oder 6 C-Atomen,
R(4) und R(5) unabhängig voneinander Wasserstoff, Methyl oder Ethyl;
R(6), R(7), R(8) und R(9) unabhängig voneinander Wasserstoff, Alkyl mit 1, 2, 3 oder 4 C-Atomen; Methoxy, Ethoxy, F, Cl, CN, NR(12)R(13), -O_{q}-(CH₂)ᵣ-CF₃ oder -(SO_{w})ₜ-(CH₂)ᵤ-CF₃;
R(12) und R(13) unabhängig voneinander Wasserstoff, Methyl oder Ethyl;
w Null, 1 oder 2;
r und u Null, 1, 2 oder 3;
q und t unabhängig voneinander Null oder 1;
oder
R(6) und R(7) oder R(7) und R(8) oder R(8) und R(9) zusammen mit dem sie tragenden Phenylring ein Naphthalin-System;
A -S-, -SO- oder -SO₂-
sowie deren pharmazeutisch verträgliche Salze.

3. Verbindungen der Formel I nach Ansprüchen 1 oder 2, in der bedeuten:
R(1) Wasserstoff, Methyl, Ethyl, Methoxy, Ethoxy, F, Cl, NR(10)R(11), -Oₚ-(CH₂)ₙ-CF₃ oder -(SOₘ)ₚ-(CH₂)ₙCF₃;
R(10) und R(11) unabhängig voneinander Wasserstoff, Methyl, Ethyl oder -CH₂-CF₃;
m Null, 1 oder 2;
n, p unabhängig voneinander Null oder 1;
R(2) Wasserstoff, F, Cl, Methyl, Cycloalkyl mit 3, 4, 5 oder 6 C-Atomen,
R(3), R(4) und R(5) Wasserstoff;
R(6), R(7), R(8) und R(9) unabhängig voneinander Wasserstoff, Methyl; Methoxy, Ethoxy, F, Cl, NR(12)R(13), -Oq-(CH₂)ᵣ-CF₃ oder -(SO_{w})ₜ-(CH₂)ᵤ-CF₃;
R(12) und R(13) unabhängig voneinander Wasserstoff, Methyl oder Ethyl;
w Null, 1 oder 2;
q, r, t und u unabhängig voneinander Null oder 1;
oder
R(6) und R(7) oder R(7) und R(8) oder R(8) und R(9) zusammen mit dem sie tragenden Phenylring ein Naphthalin-System;
A -S-, -SO- oder -SO₂-
sowie deren pharmazeutisch verträgliche Salze.

4. Verbindungen der Formel I nach Ansprüchen 1 bis 3, in der bedeuten:
R(1) Wasserstoff, Methyl, Methoxy, Ethoxy, Cl, NR(10)R(11), -O-CH₂-CF₃ oder -(SOₘ)ₚ-(CH₂)ₙ-CF₃;
R(10) und R(11) unabhängig voneinander Wasserstoff, Methyl, Ethyl oder -CH₂-CF₃;
m Null, 1 oder 2;
p Null oder 1;
R(2) Wasserstoff, F, Cl oder Methyl;
R(3), R(4) und R(5) Wasserstoff;
R(6), R(7), R(8) und R(9) unabhängig voneinander Wasserstoff, Methyl; Methoxy, Ethoxy, F, Cl, -O-CH₂-CF₃ oder -(SO_{w})ₜ-(CH₂)ᵤ-CF₃;
w Null, 1 oder 2;
t und u unabhängig voneinander Null oder 1;
oder
R(6) und R(7) oder R(7) und R(8) oder R(8) und R(9) zusammen mit dem sie tragenden Phenylring ein Naphthalin-System;
A -SO₂-
sowie deren pharmazeutisch verträgliche Salze.

5. Verwendung einer Verbindung I nach Anspruch 1 zur Herstellung eines Medikaments zu Behandlung oder Prophylaxe von durch ischämische Zustände verursachten Krankheiten.

6. Verwendung einer Verbindung I nach Anspruch 1 zur Herstellung eines Medikaments zur Behandlung oder Prophylaxe des Herzinfarkts und von Arrhythmien.

7. Verwendung einer Verbindung I nach Anspruch 1 zur Herstellung eines Medikaments zur Behandlung oder Prophylaxe der Angina Pectoris.

8. Verwendung einer Verbindung I nach Anspruch 1 zur Herstellung eines Medikaments zur Behandlung oder Prophylaxe von ischämischen Zuständen des Herzens.

9. Verwendung einer Verbindung I nach Anspruch 1 zur Herstellung eines Medikaments zur Behandlung oder Prophylaxe von ischämischen Zuständen des peripheren und zentralen Nervensystems und des Schlaganfalls.

10. Verwendung einer Verbindung I nach Anspruch 1 zur Herstellung eines Medikaments zur Behandlung oder Prophylaxe von ischämischen Zuständen peripherer Organe und Gliedmaßen.

11. Verwendung einer Verbindung I nach Anspruch 1 zur Herstellung eines Medikaments zur Behandlung von Schockzuständen.

12. Verwendung einer Verbindung 1 nach Anspruch 1 zur Herstellung eines Medikaments zum Einsatz bei chirurgischen Operationen und Organtransplantationen.

13. Verwendung einer Verbindung I nach Anspruch 1 zur Herstellung eines Medikaments zur Konservierung und Lagerung von Transplantaten für chirurgische Maßnahmen.

14. Verwendung einer Verbindung I nach Anspruch 1 zur Herstellung eines Medikaments zur Behandlung von Krankheiten, bei denen die Zellproliferation eine primäre oder sekundäre Ursache darstellt.

15. Verwendung einer Verbindung I nach Anspruch 1 zur Herstellung eines Medikaments zur Behandlung oder Prophylaxe von Störungen des Fettstoffwechsels.

16. Arzneimittel, enthaltend eine wirksame Menge einer Verbindung 1 nach einem oder mehreren der Ansprüche 1 bis 4.

## Claims

1. A dihydrothiaphenanthrenecarbonylguanidine of the formula I 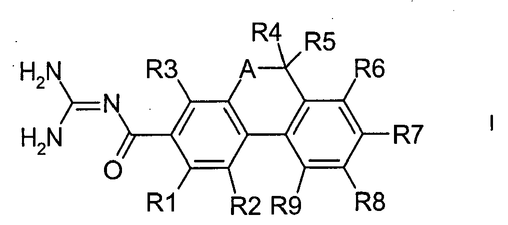 in which the meanings are:
R(1) and R(3) independently of one another hydrogen, alkyl having 1, 2, 3 or 4 carbon atoms, alkoxy having 1, 2, 3 or 4 carbon atoms, F, Cl, Br, I, CN, NR(10)R(11),
-Oₚ-(CH₂)ₙ-(CF₂)_{X}-CF₃ or -(SOₘ)ₚ-(CH₂)ₙ-(CF₂)ₓ-CF₃;
R(10) and R(11), independently of one another hydrogen, alkyl having 1, 2, 3 or 4 carbon atoms or -(CH₂)ₙ-(CF₂)ₓ-CF₃;
m zero, 1 or 2;
n zero, 1, 2, 3, 4, 5 or 6;
x and p independently of one another zero or 1;
R(2) hydrogen, F, Cl, Br, I, CN, alkyl having 1, 2, 3 or 4 carbon atoms, methoxy, cycloalkyl having 3, 4, 5, 6, 7 or 8 carbon atoms,
R(4) and R(5) independently of one another hydrogen, alkyl having 1, 2, 3 or 4 carbon atoms;
R(6), R(7), R(8) and R(9) independently of one another hydrogen, alkyl having 1, 2, 3 or 4 carbon atoms; alkoxy having 1, 2, 3 or 4 carbon atoms, F, Cl, Br, I, CN, NR(12)R(13),
-O_{q}-(CH₂)ᵣ-(CF₂)ₛ-CF₃ or -(SO_{w})ₜ-(CH₂)ᵤ-(CF₂)ᵥ-CF₃;
R(12) and R(13) independently of one another hydrogen, alkyl having 1, 2, 3 or 4 carbon atoms;
w zero, 1 or 2;
r and u zero, 1, 2, 3, 4, 5 or 6;
q, s, t and v independently of one another zero or 1;
or
R(6) and R(7) or R(7) and R(8) or R(8) and R(9) together with the phenyl ring carrying them a naphthalene system;
A -S-, -SO- or -SO₂-
and the pharmaceutically suitable salts thereof.

2. A compound of the formula I as claimed in claim 1, in which the meanings are:
R(1) and R(3) independently of one another hydrogen, methyl, ethyl, methoxy, ethoxy, F, Cl, CN, NR(10)R(11),-Oₚ-(CH₂)ₙ-CF₃ or -(SOₘ)ₚ-(CH₂)ₙ-CF₃;
R(10) and R(11) independently of one another hydrogen, methyl, ethyl or -CH₂-CF₃;
m zero, 1 or 2;
n zero, 1, 2 or 3;
p independently of one another zero or 1;
R(2) hydrogen, F, Cl, CN, alkyl having 1, 2, 3 or 4 carbon atoms, methoxy, cycloalkyl having 3, 4, 5 or 6 carbon atoms,
R(4) and R(5) independently of one another hydrogen, methyl or ethyl;
R(6), R(7), R(8) and R(9) independently of one another hydrogen, alkyl having 1, 2, 3 or 4 carbon atoms; methoxy, ethoxy, F, Cl, CN, NR(12)R(13), -O_{q}-(CH₂)ᵣCF₃ or
-(SO_{w})ₜ-(CH₂)ᵤ-CF₃;
R(12) and R(13) independently of one another hydrogen, methyl or ethyl;
w zero, 1 or 2;
r and u zero, 1, 2 or 3;
q and t independently of one another zero or 1;
or
R(6) and R(7) or R(7) and R(8) or R(8) and R(9) together with the phenyl ring carrying them a naphthalene system;
A -S-, -SO- or -SO₂-
and the pharmaceutically suitable salts thereof.

3. A compound of the formula I as claimed in claims 1 or 2, in which the meanings are:
R(1) hydrogen, methyl, ethyl, methoxy, ethoxy, F, Cl, NR(10)R(11),
-Oₚ-(CH₂)ₙ-CF₃ or -(SOₘ)ₚ-(CH₂)ₙ-CF₃;
R(10) and R(11) independently of one another hydrogen, methyl, ethyl or -CH₂-CF₃;
m zero, 1 or 2;
n, p independently of one another zero or 1;
R(2) hydrogen, F, Cl, methyl, cycloalkyl having 3, 4, 5 or 6 carbon atoms,
R(3), R(4) and R(5) hydrogen;
R(6), R(7), R(8) and R(9) independently of one another hydrogen, methyl; methoxy, ethoxy, F, Cl, NR(12)R(13), -O_{q}-(CH₂)ᵣ-CF₃ or-(SO_{w})ₜ-(CH₂)ᵤ-CF₃;
R(12) and R(13) independently of one another hydrogen, methyl or ethyl;
w zero, 1 or 2;
q, r, t and u independently of one another zero or 1;
or
R(6) and R(7) or R(7) and R(8) or R(8) and R(9) together with the phenyl ring carrying them a naphthalene system;
A -S-, -SO- or -SO₂-
and the pharmaceutically suitable salts thereof.

4. A compound of the formula I as claimed in claims 1 to 3, in which the meanings are:
R(1) hydrogen, methyl, methoxy, ethoxy, Cl, NR(10)R(11), -O-CH₂-CF₃ or -(SOₘ)ₚ-(CH₂)ₙ-CF₃;
R(10) and R(11) independently of one another hydrogen, methyl, ethyl or -CH₂-CF₃;
m zero, 1 or 2;
p zero or 1;
R(2) hydrogen, F, Cl or methyl;
R(3), R(4) and R(5) hydrogen;
R(6), R(7), R(8) and R(9) independently of one another hydrogen, methyl; methoxy, ethoxy, F, Cl, -O-CH₂-CF₃ or -(SO_{w})ₜ-(CH₂)ᵤ-CF₃;
w zero, 1 or 2;
t and u independently of one another zero or 1;
or
R(6) and R(7) or R(7) and R(8) or R(8) and R(9) together with the phenyl ring carrying them a naphthalene system;
A -SO₂-
and the pharmaceutically suitable salts thereof.

5. The use of a compound I as claimed in claim 1 for producing a medicament for the treatment or prophylaxis of disorders caused by ischemic states.

6. The use of a compound I as claimed in claim 1 for producing a medicament for the treatment or prophylaxis of myocardial infarction and of arrhythmias.

7. The use of a compound I as claimed in claim 1 for producing a medicament for the treatment or prophylaxis of angina pectoris.

8. The use of a compound I as claimed in claim 1 for producing a medicament for the treatment or prophylaxis of ischemic states of the heart.

9. The use of a compound I as claimed in claim 1 for producing a medicament for the treatment or prophylaxis of ischemic states of the peripheral and central nervous system and of stroke.

10. The use of a compound I as claimed in claim 1 for producing a medicament for the treatment or prophylaxis of ischemic states of peripheral organs and limbs.

11. The use of a compound I as claimed in claim 1 for producing a medicament for the treatment of states of shock.

12. The use of a compound I as claimed in claim 1 for producing a medicament for use during surgical operations and organ transplantations.

13. The use of a compound I as claimed in claim 1 for producing a medicament for the preservation and storage of transplants for surgical procedures.

14. The use of a compound I as claimed in claim 1 for producing a medicament for the treatment of disorders in which cell proliferation represents a primary or secondary cause.

15. The use of a compound I as claimed in claim 1 for producing a medicament for the treatment or prophylaxis of disorders of lipid metabolism.

16. A medicament comprising an effective amount of a compound I as claimed in one or more of claims 1 to 4.

## Revendications

1. Dihydro-thia-phénanthrène-carbonylguanidines de formule I 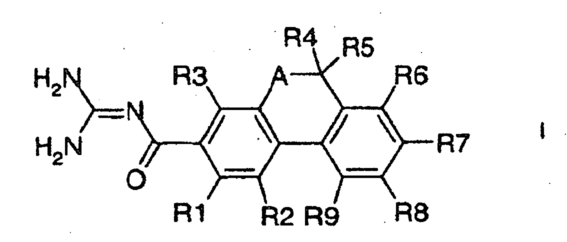 dans laquelle
R(1) et R(3) représentent, indépendamment l'un de l'autre, un atome d'hydrogène, un groupe alkyle ayant 1, 2, 3 ou 4 atomes de carbone, alcoxy ayant 1, 2, 3 ou 4 atomes de carbone, F, Cl, Br, I, CN, NR(10)R(11), -Oₚ-(CH₂)ₙ-(CF₂)ₓ-CF₃ ou -(SOₘ)ₚ-(CH₂)ₙ-(CF₂)ₓ-CF₃ ;
R(10) et R(11) représentent, indépendamment l'un de l'autre, un atome d'hydrogène, un groupe alkyle ayant 1, 2, 3 ou 4 atomes de carbone ou -(CH₂)ₙ-(CF₂)ₓ-CF₃ ;
m est zéro, 1 ou 2;
n est zéro, 1, 2, 3, 4, 5 ou 6;
x et p représentent, indépendamment l'un de l'autre, zéro ou 1 ;
R(2) représente un atome d'hydrogène, F, Cl, Br, I, CN, un groupe alkyle ayant 1, 2, 3 ou 4 atomes de carbone, méthoxy, cycloalkyle ayant 3, 4, 5, 6, 7 ou 8 atomes de carbone ;
R(4) et R(5) représentent, indépendamment l'un de l'autre, un atome d'hydrogène, un groupe alkyle ayant 1, 2, 3 ou 4 atomes de carbone ;
R(6), R(7), R(8) et R(9) représentent, indépendamment les uns des autres, un atome d'hydrogène, un groupe alkyle ayant 1, 2, 3 ou 4 atomes de carbone, alcoxy ayant 1, 2, 3 ou 4 atomes de carbone, F, Cl, Br, I, CN, NR(12)R(13), -O_{q}-(CH₂)ᵣ-(CF₂)ₛ-CF₃ ou -(SO_{w})ₜ-(CH₂)ᵤ-(CF₂)ᵥ-CF₃ ;
R(12) et R(13) représentent, indépendamment l'un de l'autre, un atome d'hydrogène, un groupe alkyle ayant 1, 2, 3 ou 4 atomes de carbone ;
w est zéro, 1 ou 2 ;
r et u représentent zéro, 1, 2, 3, 4, 5 ou 6;
q, s, t et v représentent, indépendamment les uns des autres, zéro ou 1 ;
ou
R(6) et R(7) ou R(7) et R(8) ou R(8) et R(9) forment conjointement avec le cycle phényle qui les porte un système naphtalène ;
A représente -S-, -SO- ou -SO₂-
ainsi que leurs sels pharmaceutiquement acceptables.

2. Composés de formule I selon la revendication 1, dans lesquels:
R(1) et R(3) représentent, indépendamment l'un de l'autre, un atome d'hydrogène, le groupe méthyle, éthyle, méthoxy, éthoxy, F, Cl, CN, NR(10) R(11), -Oₚ-(CH₂)ₙ-CF₃ ou -(SOₘ)ₚ-(CH₂)ₙ-CF₃ ;
R(10) et R(11) représentent, indépendamment l'un de l'autre, un atome d'hydrogène, le groupe méthyle, éthyle ou -CH₂-CF₃ ;
m est zéro, 1 ou 2 ;
n est zéro, 1, 2 ou 3 ;
p représente zéro ou 1 ;
R(2) représente un atome d'hydrogène, F, Cl, CN, un groupe alkyle ayant 1, 2, 3 ou 4 atomes de carbone, méthoxy, cycloalkyle ayant 3, 4, 5 ou 6 atomes de carbone ;
R(4) et R(5) représentent, indépendamment l'un de l'autre, un atome d'hydrogène, le groupe méthyle ou éthyle ;
R(6), R(7), R(8) et R(9) représentent, indépendamment les uns des autres, un atome d'hydrogène, un groupe alkyle ayant 1, 2, 3 ou 4 atomes de carbone, méthoxy, éthoxy, F, Cl, CN, NR(12)R(13), -O_{q}-(CH₂)ᵣ-CF₃ ou -(SO_{w})ₜ-(CH₂)ᵤ-CF₃ ;
R(12) et R(13) représentent, indépendamment l'un de l'autre, un atome d'hydrogène, le groupe méthyle ou éthyle;
w est zéro, 1 ou 2 ;
r et u représentent zéro, 1, 2 ou 3;
q et t représentent, indépendamment l'un de l'autre, zéro ou 1 ;
ou
R(6) et R(7) ou R(7) et R(8) ou R(8) et R(9) forment conjointement avec le cycle phényle qui les porte un système naphtalène;
A représente -S-, -SO- ou -SO₂-
ainsi que leurs sels pharmaceutiquement acceptables.

3. Composés de formule I selon la revendication 1 ou 2, dans lesquels :
R(1) représente un atome d'hydrogène, le groupe méthyle, éthyle, méthoxy, éthoxy, F, Cl, NR(10)R(11), -Oₚ-(CH₂)ₙ-CF₃ ou -(SOₘ)ₚ-(CH₂)ₙ-CF₃ ;
R(10) et R(11) représentent, indépendamment l'un de l'autre, un atome d'hydrogène, le groupe méthyle, éthyle ou -CH₂-CF₃ ;
m est zéro, 1 ou 2 ;
n, p représentent, indépendamment l'un de l'autre, zéro ou 1 ;
R(2) représente un atome d'hydrogène, F, Cl, un groupe méthyle, cycloalkyle ayant 3, 4, 5 ou 6 atomes de carbone ;
R(3), R(4) et R(5) représentent chacun un atome d'hydrogène;
R(6), R(7), R(8) et R(9) représentent, indépendamment les uns des autres, un atome d'hydrogène, le groupe méthyle, méthoxy, éthoxy, F, Cl, NR(12)R(13), -O_{q}-(CH₂)ᵣ-CF₃ ou -(SO_{w})ₜ-(CH₂)ᵤ-CF₃;
R(12) et R(13) représentent, indépendamment l'un de l'autre, un atome d'hydrogène, le groupe méthyle ou éthyle ;
w est zéro, 1 ou 2 ;
q, r, t et u représentent indépendamment les uns des autres, zéro ou 1 ;
ou
R(6) et R(7) ou R(7) et R(8) ou R(8) et R(9) forment conjointement avec le cycle phényle qui les porte un système naphtalène;
A représente -S-, -SO- ou -SO₂-
ainsi que leurs sels pharmaceutiquement acceptables.

4. Composés de formule I selon les revendications 1 à 3, dans lesquels :
R(1) représente un atome d'hydrogène, le groupe méthyle, méthoxy, éthoxy, Cl, NR(10)R(11), -O-CH₂-CF₃ ou -(SOₘ)ₚ-(CH₂)-CF₃ ;
R(10) et R(11) représentent, indépendamment l'un de l'autre, un atome d'hydrogène, le groupe méthyle, éthyle ou -CH₂-CF₃ ;
m est zéro, 1 ou 2 ;
p représente zéro ou 1 ;
R(2) représente un atome d'hydrogène, F, Cl ou le groupe méthyle ;
R(3), R(4) et R(5) représentent chacun un atome d'hydrogène ;
R(6), R(7), R(8) et R(9) représentent, indépendamment les uns des autres, un atome d'hydrogène, le groupe méthyle, méthoxy, éthoxy, F, Cl, -O-CH₂-CF₃ ou -(SO_{w})ₜ-(CH₂)ᵤ-CF₃ ;
w est zéro, 1 ou 2 ;
t et u représentent indépendamment l'un de l'autre, zéro ou 1;
ou
R(6) et R(7) ou R(7) et R(8) ou R(8) et R(9) forment conjointement avec le cycle phényle qui les porte un système naphtalène ;
A représente -SO₂-
ainsi que leurs sels pharmaceutiquement acceptables.

5. Utilisation d'un composé I selon la revendication 1, pour la fabrication d'un médicament destiné au traitement ou à la prophylaxie de maladies causées par des états ischémiques

6. Utilisation d'un composé I selon la revendication 1, pour la fabrication d'un médicament destiné au traitement ou à la prophylaxie de l'infarctus du myocarde et d'arythmies.

7. Utilisation d'un composé I selon la revendication 1, pour la fabrication d'un médicament destiné au traitement ou à la prophylaxie de l'angine de poitrine.

8. Utilisation d'un composé I selon la revendication 1, pour la fabrication d'un médicament destiné au traitement ou à la prophylaxie d'états ischémiques du coeur.

9. Utilisation d'un composé I selon la revendication 1, pour la fabrication d'un médicament destiné au traitement ou à la prophylaxie d'états ischémiques du système nerveux central et périphérique et de l'accident vasculaire cérébral.

10. Utilisation d'un composé I selon la revendication 1, pour la fabrication d'un médicament destiné au traitement ou à la prophylaxie d'états ischémiques des organes périphériques et des membres.

11. Utilisation d'un composé I selon la revendication 1, pour la fabrication d'un médicament destiné au traitement d'états de choc.

12. Utilisation d'un composé I selon la revendication 1, pour la fabrication d'un médicament destiné à l'utilisation dans des opérations chirurgicales et des transplantations d'organes.

13. Utilisation d'un composé I selon la revendication 1, pour la fabrication d'un médicament destiné à la conservation et au stockage de transplants pour des interventions chirurgicales.

14. Utilisation d'un composé I selon la revendication 1, pour la fabrication d'un médicament destiné au traitement de maladies dans lesquelles la prolifération cellulaire représente une cause primaire ou secondaire.

15. Utilisation d'un composé I selon la revendication 1, pour la fabrication d'un médicament destiné au traitement ou à la prophylaxie de troubles du métabolisme lipidique.

16. Médicament, contenant une quantité efficace d'un composé I selon une ou plusieurs des revendications 1 à 4.
